# DEMANDE DE BREVET EUROPEEN

(11) **EP 1 203 561 A1**
(43) Date de publication de la demande: **08.05.2002**
(21) Numéro de dépôt: 00811028.0
(22) Date de dépôt: 03.11.2000
(51) Int. Cl.: A61B 5/103, A61B 5/11, A61B 6/04

(54) **Dispositif pour mettre les genoux d'un patient sous contrainte antero-posterieure et utilisation de ce dispositif**

(71) Demandeur: Assal, Mathieu, 1207 Genève (CH)
(72) Inventeur: Assal, Mathieu, 1207 Genève (CH)
(74) Mandataire: Savoye, Jean-Paul

(57) **Abrégé**

Ce dispositif pour mettre les genoux d'un patient sous contrainte antéro-postérieure, comporte un bâti (1) portant des moyens de fixation symétriques (3a, 3b, 2a, 2b) des deux cuisses et des deux jambes à ce bâti (1), agencés pour former entre les cuisses et les jambes deux angles déterminés (γ) sensiblement égaux, un organe de contrainte (6), des moyens de guidage (7) de cet organe de contrainte (6) selon une direction sensiblement perpendiculaire aux tibias et passant au niveau des parties proximales des tibias respectifs, des moyens (11, 11a) pour déplacer ledit organe de contrainte (6) le long desdits moyens de guidage (7), des moyens (8, 12) pour mesurer et/ou limiter la pression résultante exercée sur les tibias par ledit organe de contrainte (6) et un support (4) pour positionner une plaque radiographique (5) selon un plan perpendiculaire à un faisceau (14) émis par une source de rayons X (13).

## Description

La présente invention se rapporte à un dispositif pour mettre les genoux d'un patient sous contrainte antéro-postérieure spécifique en vue de visualiser la laxité ou l'instabilité consécutive à une lésion ligamentaire et/ou capsulaire et à une utilisation de ce dispositif.

Le diagnostic des lésions ligamentaires, notamment celles du ligament croisé postérieur peut poser des problèmes et une documentation précise de la laxité du genou est essentielle à la fois du point de vue diagnostique et thérapeutique. Différentes méthodes cliniques ont été préconisées pour mesurer la laxité postérieure sous l'effet d'une traction antéro-postérieure et de la gravité notamment, encore que l'interprétation repose sur l'expérience du praticien et peut être fortement subjective. La force manuelle appliquée et l'estimation au moyen de repères anatomiques peut varier considérablement et rendre l'évaluation du déplacement difficile avec des genoux instables en direction postérieure. Différentes méthodes mécaniques ont été développées pour évaluer objectivement le déplacement sagittal. La précision de ces dispositifs externes et des arthromètres en particulier est satisfaisante dans le cas de la laxité antérieure, mais se révèle d'une valeur discutable dans l'évaluation de la laxité postérieure.

Les techniques radiologiques sont fiables pour mesurer différents types de laxités à la suite de lésions ligamentaires du genou. Dans l'évaluation de la laxité postérieure, l'examen radiologique surmonte certaines des limitations rencontrées aussi bien avec l'examen physique qu'avec les tests arthrométriques. Le déplacement squelettique seul est mesuré et des erreurs provenant de l'interposition de tissus mous sont minimisées. L'examen radiologique s'est révélé supérieur à la fois à l'arthromètre et au test clinique de traction postérieure, pour déterminer l'état du ligament croisé postérieur, lorsqu'il est réalisé dans des conditions parfaitement reproductibles. Toutefois, dans la pratique clinique courante, ces examens radiologiques sont moins fiables et plusieurs facteurs ont été identifiés comme conduisant à un diagnostic imprécis. Ces facteurs englobent la rotation tibiale inégale entre les deux genoux, des angles de flexion différents, des valeurs inégales de contraintes antéro-postérieures et de la zone d'application de la contrainte, la technique de mesure et l'identification des repères osseux en comparant les deux genoux.

On connaît déjà un appareil de mesure du déplacement postérieur du tibia utilisant une vision latérale et vendu sous la marque Telos. Cependant, on sait que plusieurs variables peuvent interférer avec la précision de cette méthode.

G. Puddu, P. Chambat, E. Gianni dans « The axial view to evaluate the posterior instability of the knee » Arthroscopy, 2000-3, recommandent d'utiliser la vue axiale pour évaluer la laxité postérieure du genou, ce que ne permet pas de faire l'appareil susmentionné. La méthode consiste en une radiographie axiale des deux genoux pliés à 70°. Par le terme axial, on entend une radiographie dont les rayons X pénètrent la rotule dans la direction de son grand axe, en formant un angle d'environ 26° avec l'axe du tibia, cet angle pouvant être compris entre 15° et 45°. La particularité de cette technique est que le déplacement sagittal est mesuré dans le plan axial. Les deux genoux sont théoriquement soumis aux mêmes conditions radiologiques, avec un degré de flexion et de rotation tibiales identiques et la même force de gravité.

L'efficacité de cette méthode est due au fait que les repères osseux sont particulièrement clairs par rapport à la vue latérale traditionnelle du genou.

Le but de la présente invention est d'apporter une solution apte à mettre en oeuvre la méthode de diagnostic par mesure de la laxité postérieure dans le plan axial.

A cet effet, la présente invention a pour objet un dispositif pour mettre les genoux d'un patient sous contrainte antéro-postérieure spécifique en vue de visualiser la laxité ou l'instabilité consécutive à une lésion ligamentaire, tel que défini par la revendication 1. Elle a également pour objet une utilisation de ce dispositif.

L'invention sera mieux comprise à la lecture de la description qui suit, relative à une forme d'exécution du dispositif objet de l'invention et illustrée, schématiquement et à titre d'exemple, par le dessin annexé dans lequel:
les figures 1a et 1b sont des dessins de vues radiographiques axiales des deux genoux d'un patient, respectivement d'un genou sain et d'un genou blessé;
la figure 2 est une vue en perspective de l'observation du déplacement postérieur du tibia;
les figures 3a et 3b sont des vues latérales du genou montrant la relation entre l'angle de flexion γ et l'angle d'observation optimal β;
la figure 4 est une vue en élévation latérale de la forme d'exécution du dispositif de mise sous contrainte;
la figure 5 est une vue selon V-V de la figure 4;
la figure 6 est une vue en élévation latérale du dispositif des figures 4 et 5 monté sur un patient.

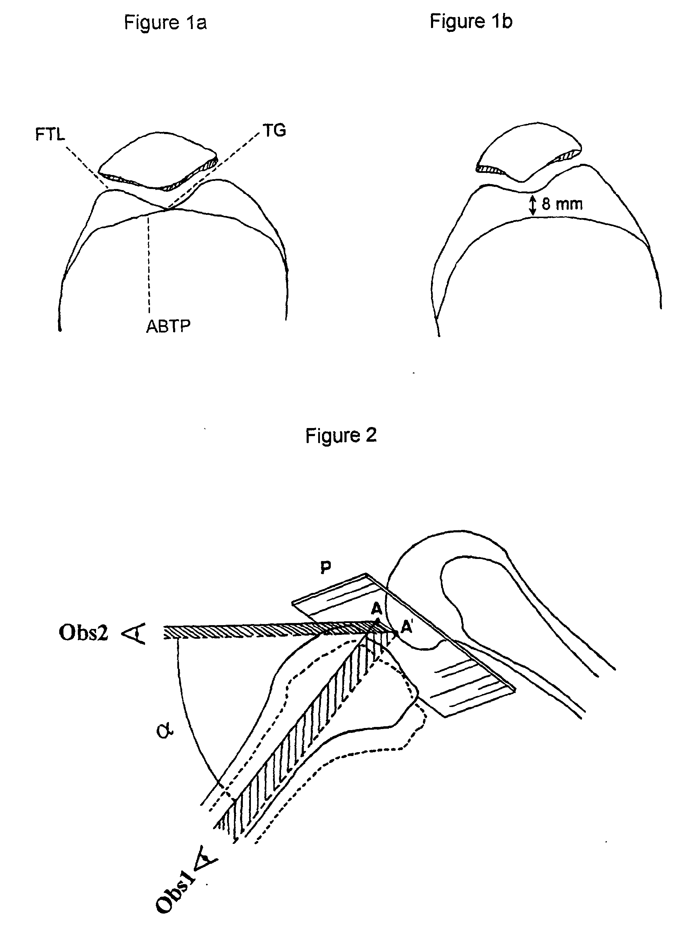

On peut identifier deux lignes sur les figures 1a, 1b, la ligne de trochlée fémorale FTL qui passe sous la rotule et la ligne du plateau tibial qui est projetée sur les condyles femoraux et représente le bord antérieur du plateau tibial ABTP. Le point le plus bas de la ligne de trochlée est la gorge de trochlée TG. La distance entre les deux lignes, de la gorge de trochlée TG au bord antérieur du plateau tibial ABTP immédiatement au-dessous est mesurée en mm sur chaque genou. La différence de mesure calculée Δ entre les deux genoux représente la valeur de translation tibiale du genou dont le ligament croisé postérieur présente une lésion.

Afin de mesurer avec précision la valeur du déplacement postérieur du tibia en vue axiale, il a fallu déterminer l'angle de flexion correct du genou ainsi que l'angle d'incidence du faisceau de rayons X, de manière qu'un déplacement observé sur la vue axiale est égal au déplacement observé sur une vue latérale.

Avec la laxité postérieure du genou, le tibia se déplace postérieurement sur le plan P constitué par les plateaux tibiaux (figure 2). En simplifiant, on peut considérer que cette surface de glissement est perpendiculaire à l'axe tibial, bien que ceci ne tienne pas compte de la pente du plateau tibial. Le déplacement peut être mesuré par la différence entre la position initiale A et la position après le déplacement postérieur A' du tibia. La distance observée entre A et A' varie selon le point d'observation (Obs1 ou un autre point tel que Obs2). Il est donc nécessaire de prendre en compte la position de l'observateur lorsque la distance réelle entre ces deux points est mesurée.

Bien que les conditions radiologiques idéales sont satisfaite lorsque le point d'observation Obs1 est aussi proche que possible de l'axe du tibia, pour des raisons pratiques, il n'est pas possible de prendre une telle vue. Par conséquent, un axe différent doit être choisi, créant ainsi un angle α avec l'axe du tibia (figure 2). On peut alors mesurer la distance réelle du déplacement postérieur A-A' en utilisant l'inverse du cosinus de cet angle. Ceci permet d'arriver à la mesure correcte du déplacement postérieur qui, autrement, est toujours sous-estimé. Par exemple, l'angle α correspond à 55°, de sorte que le facteur de correction correspond à 1,74 (1/cos 55°). Par conséquent un déplacement de 12 mm mesuré sur une radiographie vue du point d'observation Obs2 est corrigé à une valeur réelle de 21 mm (12 mm x 1,74).

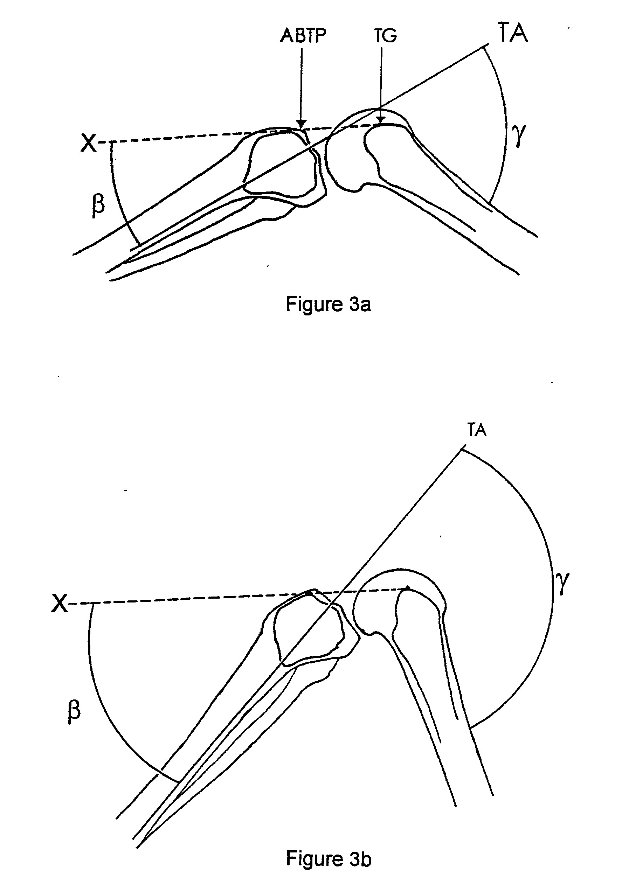

La position du bord antérieur du plateau tibial ABTP par rapport à la gorge de la trochlée TG (figure 1) varie en fonction de la flexion du genou. Comme illustré par la figure 3, lorsque le bords antérieur du plateau tibial ABTP est au même niveau que la gorge de la trochlée TG, la ligne X fait un angle (3 avec l'axe du tibia TA. Tout angle inférieur à β projetterait ABTP au-dessus de TG, ne permettant ainsi pas d'évaluer la radiographie. Pour une interprétation correcte, l'angle d'observation radiologique doit être égal ou supérieur à l'angle β. Ainsi, on peut arbitrairement appeler β « l'angle d'observation optimal ». Pour chaque angle de flexion γ, il y a un angle d'observation optimal β correspondant.

Selon Stäubli HU et al « Posterior instability of the knee near extension. » J. Bone Joint Surg. [Br] 1990;72-B:225-30 et Grood ES et al « Limits of movement in the human knee: effect of sectionning the posterior cruciate ligament and the posterolateral structures. » J. Bone Joint Surg. [Am] 1988;70-A:88-7, le déplacement postérieur du tibia avec le degré de flexion du genou atteint un maximum à 80°. C'est ainsi l'angle de flexion idéal pour mesurer la laxité postérieure.

Des testes réalisés sur un échantillon de personnes ont montré qu'avec un angle de flexion γ de 80°, l'angle d'observation β qui permet l'interprétation de la vue axiale la plus sûre est 26°. Pour cette valeur de β, le facteur de correction de l'inverse du cosinus est de 1,1 ou 10%. Cependant, le grossissement radiographique classique de 10% compense le facteur de correction ci-dessus.

En conclusion, avec un angle de flexion γ du genou de 80° et un angle β de la source de rayons X de 26°, le déplacement tibial postérieur mesuré sur la vue axiale représente la valeur de déplacement réelle et correspond avec la vue latérale vraie.

Etant donné que la précision angulaire susmentionnée est rarement obtenue dans la pratique clinique courante, il est important d'évaluer les conséquences pratiques des imprécisions dans cette technique radiologique. Deux sources d'erreurs doivent être considérées, la valeur de la flexion du genou et l'angle d'incidence du faisceau de rayons X. Selon Stäubli et al et Grood et al susmentionnés, une imprécision de l'angle de flexion affectera la valeur de la translation postérieure. Ceci peut être évité en utilisant un simple support de genou qui garantit une flexion des deux genoux de 80°. Avec une erreur de +/- 10°, le facteur d'erreur sera inférieur à 10%. Ce degré d'erreur est inférieur dans le cas d'une vue axiale à ce qu'il est avec d'autres modes d'observation qui est par exemple de +/-2 mm dans le meilleur des cas en utilisant la radiographie latérale pour mesurer le déplacement antéro-postérieur.

Un autre facteur à considérer dans l'évaluation de la laxité postérieure est celui de l'application d'une contrainte antéro-postérieure sur la partie proximale des tibias. A cet effet, des tests ont été réalisés sur 40 patients avec et sans application d'une contrainte, celle-ci étant de 90 N par genou, comme recommandé par différentes études.

Le déplacement moyen mesuré sans contrainte était de 8 mm, s'étalant entre 4-12 mm. En effectuant la mesure en appliquant une force de 180 N sur les deux tubérosités tibiales, le déplacement moyen était de 11,5 mm, s'étalant entre 6,5-18 mm. Cette étude montre l'importance d'appliquer une contrainte antéro-postérieure pour évaluer l'état du ligament croisé postérieur pcl. Sans contrainte spécifique, l'examen dépend du comportement du patient (activité des quadriceps) et/ou de l'état de contrainte des tissus souples et par conséquent, la valeur du déplacement postérieur est sensiblement sous-estimée. En plus il est important de noter qu'il n'existe pas de relation linéaire entre des radiographies exécutées avec ou sans contrainte. Notre étude a montré que pour certains patients la sous-estimation de la radiographie exécutée avec contrainte était supérieure à 100%, alors que pour d'autres elle était inférieure à 30%.

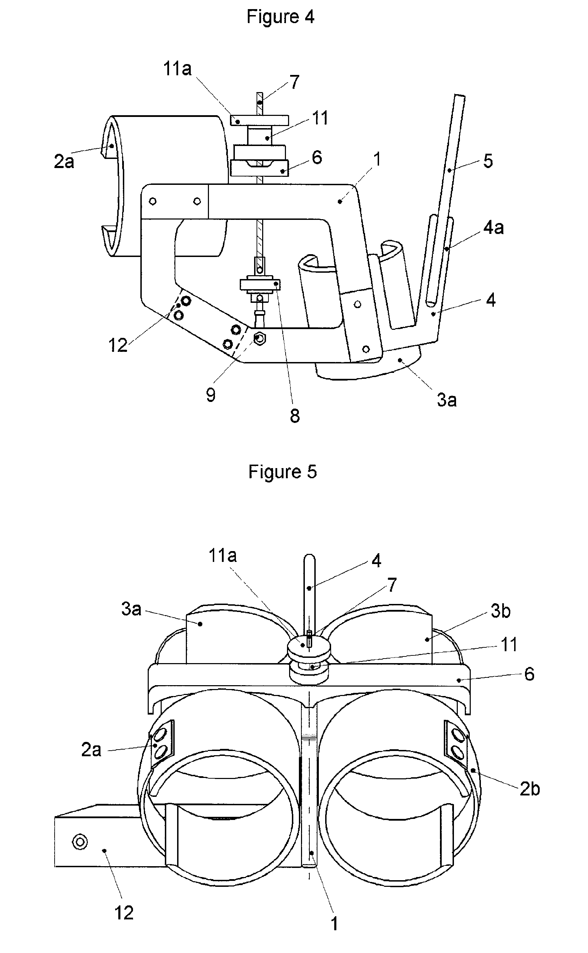

Le dispositif illustré par les figures 4 à 6 est conçu pour satisfaire aux exigences permettant de mettre en évidence la laxité antéro-postérieure d'un tibia dans le cas d'une lésion du ligament croisé postérieur.

Ce dispositif comporte un bâti formé par un cadre plat 1 destiné à se placer entre les jambes du patient à examiner. Deux sangles de fixation 2a, 2b des jambes du patient s'étendent de part et d'autre du cadre plat 1. Deux autres sangles de fixation 3a, 3b des cuisses du patient s'étendent de part et d'autre de ce même cadre plat 1. Sur la figure 4, les deux sangles 2b, 3b ont été supprimées pour permettre de voir les autres parties du dispositif qui seraient cachées autrement. Ces sangles 2a, 2b, 3a, 3b sont orientées en vue du maintien des jambes et des cuisses avec deux angles déterminés identiques, correspondant de préférence à des angles de 80° qui sont les angles d'observation optimums dont il a été question précédemment.

Ce cadre plat 1 porte un support 4 présentant une rainure de positionnement 4a d'une plaque radiographique 5. Cette rainure 4a est perpendiculaire aux deux plans sensiblement parallèles formés par les deux jambes du patient en formant les deux angles de 80° susmentionnés. Un organe de contrainte 6 des parties proximales des tibias, se présentant sous la forme d'une barre en un matériau composite formé d'une résine renforcée par des fibres de carbone, perméable aux rayons X, montée coulissante le long d'une tige de guidage 7. Cette tige de guidage 7 est, dans cet exemple, une tige filetée dont une extrémité est fixée à un côté d'une jauge de contrainte 8, dont le côté opposé est fixé à un côté du cadre plat 1 autour d'un axe d'articulation 9, destiné à permettre à la tige de guidage 7 et à la fixation de la jauge de contrainte 8 au cadre plat 1 de s'auto-aligner pour éviter d'induire des contraintes parasites faussant la valeur mesurée par cette jauge de contrainte 8. Cette jauge de contrainte 8 est, dans cet exemple, une jauge de mesure en traction-compression de type Omegadyne, LC-703150® . La tige de guidage 7 traverse avec jeu une ouverture 10 ménagée à travers un côté du cadre plat 1 opposé à celui sur lequel elle est articulée par l'intermédiaire de la jauge de contrainte 8.

La partie de la tige de guidage 7 située à l'extérieur du cadre plat 1 porte un écrou de serrage 11 solidaire d'une manette de préhension manuelle 11a, en prise avec le filetage de la tige de guidage 7. L'organe de contrainte 6 est disposé entre l'écrou de serrage 11 et le cadre plat 1, de sorte que l'écrou de serrage peut le déplacer le long de la tige de guidage 7 en direction de l'extrémité articulée au cadre plat 1.

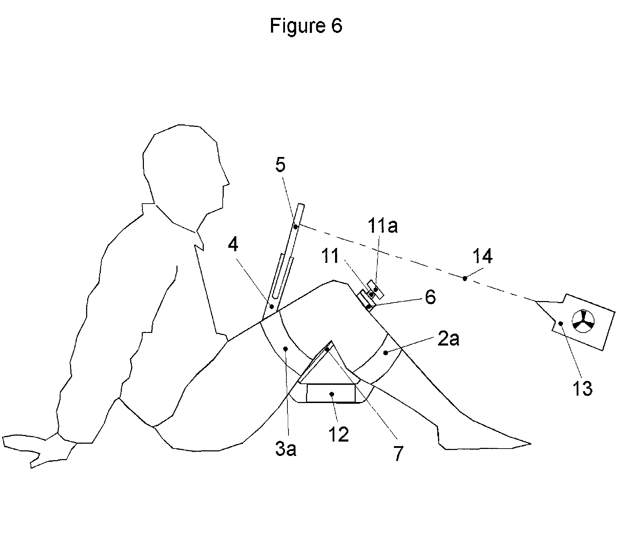

Avantageusement, la jauge de contrainte 8 est connectée à un boîtier électronique 12 pour le calcul et l'affichage de la contrainte mesurée lorsque l'organe de contrainte 6 est appliqué contre les parties proximales des tibias et soumet ces tibias à une contrainte antéro-postérieure sous la pression exercée par l'écrou de serrage 11, comme on peut le voir sur la figure 6.

Selon une variante non représentée, la jauge de contrainte 8 et le boîtier électronique 12 peuvent être remplacés par un simple limiteur de couple disposé entre la manette de préhension 11a et l'écrou de serrage 11, de manière à rompre la liaison entre cette manette 11a et l'écrou de serrage 11 dès que le couple à appliquer sur la manette de serrage 11a dépasse une valeur déterminée, fonction de la contrainte à appliquer sur les tibias par l'intermédiaire de l'organe de contrainte 6.

La figure 6 montre encore une source de rayons X 13 disposée pour que le faisceau 14 émis par cette source soit perpendiculaire à la plaque radiographique 5. Donc, ce faisceau est d'orientation générale parallèle aux deux plans formés par les deux jambes positionnées par le dispositif de mise sous contrainte des tibias selon l'invention, permettant d'obtenir sur la plaque radiographique 5, une projection correspondant à la vue axiale désirée des deux genoux. L'inclinaison du support 4 de la plaque radiographique est choisie pour qu'elle soit perpendiculaire aux rayons X et pour que l'angle formé avec l'axe du tibia soit de 26°. La source radiologique (source de rayons X) est disposée, dans cet exemple, à 110 cm de la plaque de radiologie 5.

## Revendications

1. Dispositif pour mettre les genoux d'un patient sous contrainte antéro-postérieure spécifique en vue de visualiser la laxité ou l'instabilité consécutive à une lésion ligamentaire et/ou capsulaire, **caractérisé en ce qu'**il comporte un bâti (1) portant des moyens de fixation symétriques (3a, 3b) des deux cuisses du patient à ce bâti (1), des moyens de fixation symétriques (2a, 2b) de ses deux jambes à ce bâti (1), ces moyens de fixation (2a, 2b, 3a, 3b) étant agencés pour former entre les cuisses et les jambes respectives deux angles déterminés (γ) sensiblement égaux, un organe de contrainte (6) des parties proximales des tibias respectifs, perméable aux rayons X, des moyens (7, 11, 11a) pour appliquer à cet organe de contrainte (6) une force antéro-postérieure dirigée sensiblement perpendiculairement auxdites parties proximales des tibias respectifs, des moyens (8, 12) pour mesurer et/ou limiter la pression résultante exercée sur les tibias par ledit organe de contrainte (6) et un support (4) pour positionner une plaque radiographique (5) selon un plan sensiblement perpendiculaire aux plans sensiblement parallèles contenant lesdits angles déterminés (γ) et perpendiculaire à un faisceau (14) émis par une source de rayons X (13) pour former une image axiale des genoux du patient.

2. Dispositif selon la revendication 1, **caractérisé en ce que** ledit organe de contrainte est en prise avec des moyens de guidage allongés (7) dont une extrémité est solidaire dudit bâti (1) et que ces moyens de guidage (7) sont conformés pour venir en prise avec un organe d'entraînement (11) dudit organe de contrainte (6), disposé entre ladite extrémité desdits moyens de guidage allongés (7) solidaire dudit bâti (1) et ledit organe d'entraînement (11).

3. Dispositif selon la revendication 2, **caractérisé en ce que** l'extrémité de lesdits moyens de guidage (7) solidaire dudit bâti (1) est reliée à celui-ci par l'intermédiaire d'une jauge de contrainte (8).

4. Dispositif selon la revendication 2, **caractérisé en ce qu'**un limiteur de couple est interposé entre une partie externe de préhension (11a) dudit organe d'entraînement et sa partie interne (11) en prise avec lesdits moyens de guidage (7).

5. Dispositif selon la revendication 3, **caractérisé en ce qu'**il comporte un dispositif d'affichage (12) relié à ladite jauge de contrainte (8) pour afficher la valeur de contrainte mesurée.

6. Dispositif selon l'une des revendications 2 à 5, **caractérisé en ce que** ledit bâti (1) est formé par un cadre fermé, que ladite extrémité desdits moyens de guidage (7), solidaire dudit bâti (1) est articulée (9) à un côté dudit cadre et que ces moyens de guidage (7) traversent ledit cadre et sortent par une ouverture de guidage (10) ménagée dans un côté opposé de ce cadre, ledit organe de contrainte (6) étant en prise avec la partie desdits moyens de guidage (7) située à l'extérieur dudit cadre (1).

7. Dispositif selon l'une des revendications précédentes, **caractérisé en ce que** ledit organe de contrainte (6) transparent aux rayons X est formé par un matériau composite comprenant une résine renforcée de fibre de carbone.

8. Utilisation du dispositif selon l'une des revendications précédentes, pour effectuer une radiographie axiale des deux genoux d'un patient dont les tibias sont mis sous contrainte antéro-postérieure.
